(19) Europäisches Patentamt
European Patent Office
Office européen des brevets

(11) EP 3 072 400 B1

(12) **EUROPEAN PATENT SPECIFICATION**

(45) Date of publication and mention of the grant of the patent:
**04.10.2017 Bulletin 2017/40**

(51) Int Cl.:
***A23L 2/58*** (2006.01) ***A23L 5/40*** (2016.01)
***A23L 5/42*** (2016.01) ***C09B 61/00*** (2006.01)

(21) Application number: **15161114.2**

(22) Date of filing: **26.03.2015**

(54) **NEW COLOR FOR BEVERAGES**

NEUE FARBE FÜR GETRÄNKE

NOUVELLE COULEUR POUR BOISSONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.09.2016 Bulletin 2016/39**

(73) Proprietor: **DSM IP Assets B.V.**
**6411 TE Heerlen (NL)**

(72) Inventors:
- **GRASS, Hansjoerg**
  **4303 Kaiseraugst (CH)**
- **HITZFELD, Andrea**
  **4303 Kaiseraugst (CH)**

(74) Representative: **Steck, Melanie**
**DSM Nutritional Products Ltd**
**Patent Department**
**Wurmisweg 576**
**4303 Kaiseraugst (CH)**

(56) References cited:
**EP-A1- 1 967 081** **EP-A1- 2 644 041**
**WO-A1-2008/110225** **US-A- 3 928 455**
**US-A1- 2008 113 076**

- **NELIS H J ET AL: "MICROBIAL SOURCES OF CAROTENOID PIGMENTS USED IN FOODS AND FEEDS", JOURNAL OF APPLIED BACTERIOLOGY, BLACKWELL PUBLISHING LTD., OXFORD, GB, vol. 70, no. 3, 1 January 1991 (1991-01-01), pages 181-191, XP009010863, ISSN: 0021-8847**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention is directed to a beverage comprising a milled rhodoxanthin form, wherein the milled rhodoxanthin in the form has an average particle size D(v,0.5) of the rhodoxanthin in the range of from 400 to 650 nm, more preferably in the range of from 500 nm to 600 nm, measured by Laser Diffraction; Malvern Mastersizer 3000, MIE volume distribution. Surprisingly such beverages have a color shade of rosé (diluted) to purple (concentrated) which could not be expected since rhodoxanthin itself has a red color.

When the color of such beverage is measured at the CIELAB color scale it has a color value b* in the range of from -7.5 to 0, preferably it has a color value b* in the range of from -2.5 to 0, more preferably it has a color value b* in the range of from -2.0 to -0.5. The color value h of such beverages is preferably in the range of from 350 to360, more preferably it is in the range of from 352 to 356, most preferably it is in the range of from 353 to 355.

Preferably the milled rhodoxanthin is added to the beverage as a dispersion. More preferably the milled rhodoxanthin in such a dispersion is encapsulated in a matrix of modified food starch. The present invention is therefore also directed to such dispersions and other forms of milled rhodoxanthin with the particle size as given above and to the manufacture of such milled forms, especially such milled dispersions. EP 2 644 041 A1 discloses a clear liquid formulation comprising at least one carotenoid, at least one modified food starch, at least one saccharide, and water.

## Rhodoxanthin

Rhodoxanthin (compound of formula I) can be obtained from a natural source, by fermentation or by chemical synthesis. A natural source might be conifers, e.g. plants of Taxus baccata, or Aloe sp. (see e.g. Merzlyak et al., Photochem Photobiol Sci 2005, 4, 333-340). Chemical syntheses are e.g. described in EP-A 077 439 and EP-A 085 763.

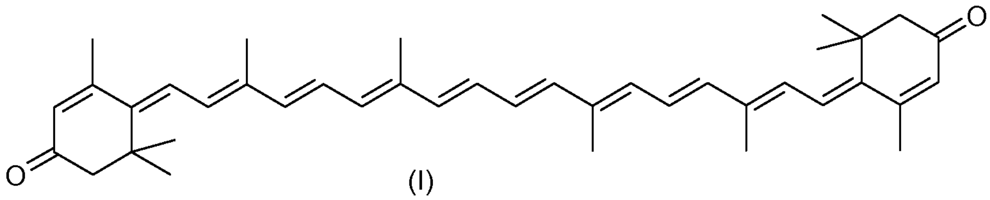

(I)

The term "rhodoxanthin" used herein encompasses the (all-E)-isomer as well as mono-, oligo- or poly-(Z)-isomers. A preferred isomer mixture contains (all-E)-rhodoxanthin, (6Z)-rhodoxanthin and (6Z,6'Z)-rhodoxanthin.

## Beverages

According to the present invention the milled rhodoxanthin form with the particle size as given above can be preferably used to color the following beverages: soft drinks as well as flavored waters, fortified waters, sports drinks, mineral drinks and carbonated beverages. Fruit juices and fruit-juice containing soft drinks may also be colored. Alcoholic beverages, instant beverage powders, sugar-containing beverages and diet beverages containing non-calorific or artificial sweeteners represent still further examples of beverages which can be colored by the rhodoxanthin form of the present invention. Such colored beverages are also encompassed by the present invention, whereby flavored waters, soft drinks and sport drinks are preferred.

Advantageously the beverages containing the milled form (preferably the milled dispersion) according to the present invention are color stable. "Color-stable" in the context of the present invention means that the color difference DE* between the initial color and the color after a storage time of 3 months should be lower than 10 (DE* < 10). A DE* < 10 means that the color difference is in the acceptable area and under DE*<3 cannot be seen by naked eyes, i.e. without the use of an apparatus such as a colorimeter.

## Amount of rhodoxanthin in the beverage

Preferably the amount of milled rhodoxanthin in the beverage is in the range of from 1 ppm to 20 ppm, more preferably it is in the range of from 1 ppm to 15 ppm, most preferably it is in the range of from 1 ppm to 10 ppm, based on the total weight of the beverage.

Preferably the rhodoxanthin is added to the beverage as a milled dispersion. Such a milled dispersion will be described in more detail below.

Milled rhodoxanthin dispersion

The liquid of such a dispersion according to the present invention is water.

The average particle size D (v,0.5) of the milled rhodoxanthin in such dispersion is preferably in the range of from 400 nm to 650 nm, more preferably in the range of from 500 nm to 600 nm, measured by Laser Diffraction; Malvern Mastersizer 3000, MIE volume distribution.

When the color of such milled dispersion is measured at the CIELAB color scale it has a color value b* in the range of from -7.5 to 0, preferably it has a color value b* in the range of from -2.5 to 0, more preferably it has a color value b* in the range of from -2.0 to -0.5. The color value h of such milled dispersion is preferably in the range of from 350 to360, more preferably it is in the range of from 352 to 356, most preferably it is in the range of from 353 to 355.

In the dispersion according to the present invention the milled rhodoxanthin is preferably embedded in a matrix of a modified food starch. The amount of the milled rhodoxanthin in the dispersion is usually in the range of from 1 to 15 weight-%, based on the total weight of the dispersion. The modified food starch (esp. OSA starch) and mixtures thereof are described in more detail below.

Additionally one or more water- and/or fat-soluble antioxidants may be present, preferably in an amount of from 0.5 to 5 weight-% in total, based on the total amount of the dispersion.

A preferred example of such water-soluble antioxidants is sodium ascorbate.

A preferred example of such fat-soluble antioxidants is dl-alpha-tocopherol.

Especially preferred is a dispersion where the milled rhodoxanthin is embedded in a matrix of modified food starch, whereby glycerine or a saccharide is added. An especially preferred example of such a dispersion is described in the examples.

Milled rhodoxanthin dispersion comprising milled rhodoxanthin, modified food starch, glycerine, water and optionally (a) water- and/or fat-soluble antioxidant/s

When glycerine is present, the amounts of water and glycerine are preferably both in the range of from 30 to 40 weight-%, based on the total weight of the dispersion, and the amount of modified food starch is preferably in the range of from 10 to 25 weight-%, based on the total weight of the dispersion, whereby the amounts of milled rhodoxanthin, modified food starch, glycerine, water and, if present, water- and/or fat-soluble antioxidants all sum up to 100 weight-%.

Milled rhodoxanthin dispersion comprising milled rhodoxanthin, modified food starch, water, saccharide and optionally (a) water- and/or fat-soluble antioxidant/s

When a saccharide is present, the amount of the saccharide is preferably in the range of from 2 to 65 weight-%, the amount of modified food starch is preferably in the range of from 15 to 45 weight-%, and the amount of water is preferably in the range of from 5 to 50 weight-%, all amounts being based on the total weight of the dispersion, whereby the amounts of milled rhodoxanthin, modified food starch, saccharide, water and, if present, water-and/or fat-soluble antioxidants all sum up to 100 weight-%.

The term "a saccharide" encompasses one saccharide or more.

The term "saccharide" in the context of the present invention encompasses mono-, di-, oligo- and polysaccharides, as well as any mixtures thereof.

Examples of monosaccharides are fructose, glucose (= dextrose), mannose, galactose, sorbose, as well as any mixtures thereof.

Preferred monosaccharides are glucose and fructose, as well as any mixture thereof.

The term "glucose" in the context of the present invention does not only mean the pure substance, but also a glucose syrup with a DE $\geq$ 90. This also applies for the other monosaccharides.

The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

Examples of disaccharides are saccharose, isomaltose, lactose, maltose and nigerose, as well as any mixture thereof.

An example of an oligosaccharide is maltodextrin.

An example of a polysaccharide is dextrin.

An example of a mixture of mono- and disaccharides is invert sugar (glucose + fructose + saccharose).

Mixtures of mono- and polysaccharides are e.g. commercially available under the tradenames Glucidex IT 47 (from Roquette Frères), Dextrose Monohydrate ST (from Roquette Frères), Sirodex 331 (from Tate & Lyle), Glucamyl F 452 (from Tate & Lyle) and Raftisweet I 50/75/35 (from Lebbe Sugar Specialties).

The most preferred saccharides are a glucose syrups or invert sugar syrups.

Other milled rhodoxanthin forms according to the present invention

Instead of a dispersion also solid forms may be used. These can be easily produced e.g. by spray-drying the dispersion which contains a saccharide such as preferably a glucose syrup or an invert sugar syrup.
Such solid forms can then also be added to instant beverage powders.

"Modified food starch"

A modified food starch is a food starch that has been chemically modified by known methods to have a chemical structure which provides it with a hydrophilic and a lipophilic portion. Preferably the modified food starch has a long hydrocarbon chain as part of its structure (preferably C5-C18).
At least one modified food starch is preferably used to make a formulation of this invention, but it is possible to use a mixture of two or more different modified food starches in one formulation.
Starches are hydrophilic and therefore do not have emulsifying capacities. However, modified food starches are made from starches substituted by known chemical methods with hydrophobic moieties. For example starch may be treated with cyclic dicarboxylic acid anhydrides such as succinic anhydrides, substituted with a hydrocarbon chain (see O. B. Wurzburg (editor), "Modified Starches: Properties and Uses, CRC Press, Inc. Boca Raton, Florida, 1986, and subsequent editions). A particularly preferred modified food starch of this invention has the following formula (I)

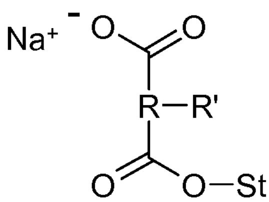

(I)

wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred compound of formula (I) is an "OSA-starch" (starch sodium octenyl succinate). The degree/extent of substitution, i.e. the number of esterified hydroxyl groups to the number of free non-esterified hydroxyl groups usually varies in a range of from 0.1% to 10%, preferably in a range of from 0.5% to 4%, more preferably in a range of from 3% to 4%.
The term "OSA-starch" denotes any starch (from any natural source such as corn, waxy maize, waxy corn, wheat, tapioca and potato or synthesized) that was treated with octenyl succinic anhydride (OSA). The degree/extent of substitution, i.e. the number of hydroxyl groups esterified with OSA to the number of free non-esterified hydroxyl groups usually varies in a range of from 0.1% to 10%, preferably in a range of from 0.5% to 4%, more preferably in a range of from 3% to 4%. OSA-starches are also known under the expression "modified food starch".
The term "OSA-starches" encompasses also such starches that are commercially available e.g. from National Starch/Ingredion under the tradenames HiCap 100, Capsul, Capsul HS, Purity Gum 2000, Clear Gum Co03, UNI-PURE, HYLON VII; from National Starch/Ingredion and Roquette Frères, respectively; from CereStar under the tradename C*EmCap or from Tate & Lyle.
In an embodiment of the present invention a commercially available modified food starch such as e.g. HiCap 100 (from National Starch/Ingredion) and ClearGum Co03 (from Roquette Frères) is used.
As already stated above dispersions that contain either glycerine or a saccharide (preferably a glucose syrup or an invert sugar syrup) are preferred. Thus, their process for manufacture is now described.

**Processes of the present invention**

The present invention is also directed to a process for the manufacture of a dispersion as described above comprising the following steps:

a) Providing a dispersion comprising crystalline rhodoxanthin, modified food starch, water and glycerine or a saccharide;
b) Milling the dispersion as obtained in step a) until the milled rhodoxanthin in the dispersion has an average particle size D(v,0.5) in the range of from 400 to 650 nm, more preferably in the range of from 500 nm to 600 nm, measured by Laser Diffraction; Malvern Mastersizer 3000, MIE volume distribution.

The steps are now described in detail below.

Step a)

The amounts of the milled rhodoxanthin, modified food starch, water and glycerine or saccharide are chosen in such a way that a dispersion results with the preferred weight percentages as given above.

Step b)

The milling may be carried out with any device known to the person skilled in the art such as colloid mills and ball mills.

The invention is now further illustrated in the following non-limiting examples.

Examples

Example 1: Manufacture of a milled rhodoxanthin dispersion according to the present invention

109.1 g of modified food starch (Capsul HS) and 202.6 g of glycerine were dissolved at 60°C in 202.6 g of water. To this solution 30 g of crystalline rhodoxanthin and 1.1 g of dl-apha-tocopherol were added.

The resulting coarse aqueous rhodoxanthin dispersion has then been milled by passing it continuously through the milling chamber of the Dispermate SL 603 agitated ball mill until the desired particle size (approx. 600 nm (average value)) has been achieved ("so called wet milling process"). The physical properties of the resulting rhodoxanthin dispersion are listed in the following:

Content of milled rhodoxanthin in the dispersion determined by HPLC = 5.4%
Content of milled rhodoxanthin in the dispersion determined by UV = 5.1 %

$$E1/1_{corr.} \text{ in } H_2O\ (\lambda_{max}) = 400\ (498\ nm)$$

The color intensity E1 /1 is the absorbance of a 1% solution and a thickness of 1 cm and is calculated as follows: E1/1 = (Amax-A650)*dilution factor / (weight of sample * content of product form in %).

"(Amax-A650)" means the value you get when you substract the Adsorption value measured at 650 nm ("A650") wavelength from the value ("Amax") that was measured at the maximum Adsorption in the UV-Spectrophotometer. "*" means "multiplied with".
"dilution factor" = the factor by which the solution has been diluted.
"weight of sample" = the amount/weight of the formulation that was used in [g]
"content of product form in %" = "the amount of milled rhodoxanthin in the dispersion in %" which is 5.1 in the present case.

Color values:

Measured as 5 ppm dispersion in $H_2O$ (1 cm, TTRAN): L*/a*/b* = 76/13/-1.3;

$$L^*/C^*/h = 76/13/354.$$

Measured as 10 ppm dispersion in $H_2O$ (1 cm, TTRAN): L*/a*/b* = 59/21/-2.1;

$$L^*/C^*/h = 59/21/354.$$

Preparation of the soft drinks

The soft drinks had the following composition:

| | Ingredient | Amount of ingredient |
|---|---|---|
| 1 | Potassium sorbate | 0.2 g |

(continued)

| | Ingredient | Amount of ingredient |
|---|---|---|
| 2 | Sugar syrup (64° Brix) | 156.2 g |
| | Ascorbic acid | 0.2 g |
| | Aqueous 50-weight-% citric acid | 5.0 g |
| | Apricot flavor (water-soluble, Givaudan 78848-56) | 0.2 g |
| | Stock solution * | 10 g (i.e. 10 ppm) |
| 3 | Water | Filled up so that a total amount of the soft drink of 1000 ml results |
| | Total amount | 1000 ml |
| * From the milled dispersion according to example 1 a stock solution was prepared, whereby the milled dispersion was diluted with water so that the stock solution had a concentration of the rhodoxanthin of 0.1 weight-% (= 1000 ppm). | | |

The soft drinks were prepared as follows:

Potassium sorbate 1) was dissolved in water, the other ingredients 2) were added one after the other while the mixture was gently stirred. Then the resulting soft drink syrup was diluted with drink water 3) in such an amount to result in 1000 ml of the soft drink. The pH of the soft drinks was in the range of 2.8 to 3.5.

5 soft drinks were prepared with the following different amounts of milled rhodoxanthin: 2 ppm, 4 ppm, 6 ppm, 8 ppm and 10 ppm.

The soft drinks were then filled in glass bottles and the bottles sealed with a metallic cap. Some of these bottles were pasteurized and some not. A tunnel pasteurizer from Mile was used for pasteurization with a holding temperature of 80°C for 1 minute at a core of the bottle.

The bottles were stored at room temperature (temperature in the range of 18 to 27°C) and under light exposure 12 hours per day with 800 Lux. Color measurements were performed directly after beverage preparation (time = 0), as well as after a storage time of 2 weeks, 30 days, 60 days and 90 days.

All samples showed a color stability as defined above.

Color measurement

The color (lightness, Chroma, and hue) of the soft drinks was determined with a HunterLab Ultra Scan Pro spectrocolorimeter (Hunter Associates Laboratory, Reston, VA,USA) and expressed on basis of the CIELAB color scale. The mode used was "TTRAN" which stands for **t**otal **tran**smission. Color measurements are carried out after CIE guidelines (Commission International d'Eclairage). Values can be expressed as planar coordinates as L*, a*, b* with L* being the measuring values for lightness, with a* being the value on the red-green axes and b* being the value on the yellow-blue axes.

The Chroma (C*) sometimes called saturation describes the vividness or dullness of a color which can be calculated as followed:

$$C^*=\surd(a^{*2}+b^{*2})$$

The angle called hue (h) describes how we perceive an object's color and can be calculated as followed:

$$h=\tan(b/a)^{(-1)}$$

**Results**

| Concentration of milled rhodoxanthin in the soft drink | 2 ppm | 4 ppm | 6 ppm | 8 ppm | 10 ppm |
|---|---|---|---|---|---|
| L* | 90.29 | 81.29 | 72.73 | 65.17 | 58.52 |
| a* | 5.42 | 10.25 | 14.67 | 18.4 | 21.08 |
| b* | -0.55 | -1.10 | -1.48 | -1.78 | -2.48 |
| C* | 5.44 | 10.31 | 14.74 | 18.49 | 21.22 |
| h | 354.24 | 353.87 | 354.22 | 354.46 | 353.29 |

## Claims

1. A beverage comprising a milled rhodoxanthin form with an average particle size D(v,0.5) of the milled rhodoxanthin in the form in the range of from 400 to 600 nm, more preferably in the range of from 500 nm to 600 nm, whereby the average particle size is measured by Laser Diffraction; Malvern Mastersizer 3000, MIE volume distribution.

2. The beverage according to claim 1, wherein the beverage has a color value b* in the range of from -7.5 to 0 at the CIELAB color scale, preferably a color value b* in the range of from -2.5 to 0 at the CIELAB color scale, more preferably a color value b* in the range of from -2.0 to - 0.5. at the CIELAB color scale.

3. The beverage according to claim 1 and/or 2, wherein the beverage has a color shade h in the range of from 350 to 360 at the CIELAB color scale, preferably a color shade h in the range of from 352 to 356 at the CIELAB color scale, more preferably a color shade h in the range of from 353 to 355 at the CIELAB color scale.

4. The beverage according to any one or more of the preceding claims, wherein the amount of milled rhodoxanthin is in the range of from 1 ppm to 20 ppm, preferably in the range of from 1 to 15 ppm, more preferably in the range of from 1 to 10 ppm, based on the total weight of the beverage.

5. The beverage according to any one or more of the preceding claims, wherein the beverage is selected from the group consisting of soft drinks, flavored waters, fortified waters, sports drinks, mineral drinks, carbonated beverages, fruit juices, fruit-juice containing soft drinks, alcoholic beverages, sugar-containing beverages and diet beverages.

6. The beverage according to any one or more of the preceding claims, wherein the milled rhodoxanthin is added to the beverage as a dispersion wherein the milled rhodoxanthin is encapsulated in a matrix of modified food starch.

7. A form comprising milled rhodoxanthin, wherein the milled rhodoxanthin in the form has an average particle size in the range of from 400 to 650 nm, more preferably in the range of from 500 nm to 600 nm, measured by Laser Diffraction; Malvern Mastersizer 3000, MIE volume distribution.

8. The form according to claim 7 being a dispersion.

9. The form according to claim 7 and/or 8, wherein the rhodoxanthin is encapsulated in a matrix of modified food starch.

10. The form according to claim 9, further comprising either glycerine or a saccharide.

11. A process for the manufacture of a dispersion according to any one or more of claims 8 to 10 comprising the following steps:

    a) Providing a dispersion comprising crystalline rhodoxanthin, modified food starch, water and glycerine or a saccharide;
    b) Milling the dispersion as obtained in step a) until the milled rhodoxanthin in the dispersion has an average particle size D(v,0.5) in the range of from 400 to 650 nm, more preferably in the range of from 500 nm to 600 nm, measured by Laser Diffraction; Malvern Mastersizer 3000, MIE volume distribution.

12. Use of a milled rhodoxanthin form with an average particle size D(v,0.5) of the milled rhodoxanthin in the form in the range of from 400 to 600 nm, more preferably in the range of from 500 nm to 600 nm, whereby the average

particle size is measured by Laser Diffraction; Malvern Mastersizer 3000, MIE volume distribution, for coloring beverages.

**13.** The use according to claim 12, wherein the milled rhodoxanthin form is a dispersion, wherein the rhodoxanthin is encapsulated in a matrix of modified food starch, and wherein the dispersion further comprises either glycerine or a saccharide.

**Patentansprüche**

**1.** Getränk, umfassend eine gemahlene Rhodoxanthinform mit einer mittleren Partikelgröße D(v,0,5) des gemahlenen Rhodoxanthins in der Form in dem Bereich von 400 bis 600 nm, bevorzugter in dem Bereich von 500 nm bis 600 nm, wobei die mittlere Partikelgröße durch Laserdiffraktion; Malvern Mastersizer 3000, MIE-Volumenerteilung, gemessen ist.

**2.** Getränk gemäß Anspruch 1, wobei das Getränk einen Farbwert b* in dem Bereich von -7,5 bis 0 auf der CIELAB-Farbskala, vorzugsweise einen Farbwert b* in dem Bereich von -2,5 bis 0 auf der CIELAB-Farbskala, bevorzugter einen Farbwert b* in dem Bereich von -2,0 bis -0,5 auf der CIELAB-Farbskala, aufweist.

**3.** Getränk gemäß Anspruch 1 und/oder Anspruch 2, wobei das Getränk einen Farbton h in dem Bereich von 350 bis 360 auf der CIELAB-Farbskala, vorzugsweise einen Farbton h in dem Bereich von 352 bis 356 auf der CIELAB-Farbskala, bevorzugter einen Farbton h in dem Bereich von 353 bis 355 auf der CIELAB-Farbskala, aufweist.

**4.** Getränk gemäß einem oder mehreren der vorstehenden Ansprüche, wobei die Menge an gemahlenem Rhodoxanthin in dem Bereich von 1 ppm bis 20 ppm, vorzugsweise in dem Bereich von 1 bis 15 ppm, bevorzugter in dem Bereich von 1 bis 10 ppm, bezogen auf das Gesamtgewicht des Getränks, liegt.

**5.** Getränk gemäß einem oder mehreren der vorstehenden Ansprüche, wobei das Getränk ausgewählt ist aus der Gruppe bestehend aus Softdrinks, aromatisiertem Wasser, verstärktem Wasser, Sportgetränken, Mineralstoffgetränken, kohlensäurehaltigen Getränken, Fruchtsäften, fruchtsafthaltigen Softdrinks, alkoholischen Getränken, zuckerhaltigen Getränken und Diätgetränken.

**6.** Getränk gemäß einem oder mehreren der vorstehenden Ansprüche, wobei das gemahlene Rhodoxanthin als Dispersion zu dem Getränk zugegeben wird, wobei das gemahlene Rhodoxanthin in einer Matrix aus modifizierter Lebensmittelstärke verkapselt ist.

**7.** Form, umfassend gemahlenes Rhodoxanthin, wobei das gemahlene Rhodoxanthin in der Form eine mittlere Partikelgröße in dem Bereich von 400 bis 650 nm, bevorzugter in dem Bereich von 500 nm bis 600 nm, aufweist, gemessen durch Laserdiffraktion; Malvern Mastersizer 3000, MIE-Volumenerteilung.

**8.** Form gemäß Anspruch 7, die eine Dispersion ist.

**9.** Form gemäß Anspruch 7 und/oder 8, wobei das Rhodoxanthin in einer Matrix aus modifizierter Lebensmittelstärke verkapselt ist.

**10.** Form gemäß Anspruch 9, ferner umfassend entweder Glycerin oder ein Saccharid.

**11.** Verfahren zum Herstellen einer Dispersion gemäß einem oder mehreren der Ansprüche 8 bis 10, umfassend folgende Schritte:

a) Bereitstellen einer Dispersion, die kristallines Rhodoxanthin, modifizierte Lebensmittelstärke, Wasser und Glycerin oder ein Saccharid umfasst;
b) Mahlen der bei Schritt a) erhaltenen Dispersion, bis das gemahlene Rhodoxanthin in der Dispersion eine mittlere Partikelgröße D(v,0,5) in dem Bereich von 400 bis 650 nm, bevorzugter in dem Bereich von 500 nm bis 600 nm, aufweist, gemessen durch Laserdiffraktion; Malvern Mastersizer 3000, MIE-Volumenerteilung.

**12.** Verwendung einer gemahlenen Rhodoxanthinform mit einer mittleren Partikelgröße D(v,0,5) des gemahlenen Rhodoxanthins in der Form in dem Bereich von 400 bis 600 nm, bevorzugter in dem Bereich von 500 nm bis 600 nm,

wobei die mittlere Partikelgröße durch Laserdiffraktion; Malvern Mastersizer 3000, MIE-Volumenerteilung, gemessen ist, zum Färben von Getränken.

**13.** Verwendung gemäß Anspruch 12, wobei die gemahlene Rhodoxanthinform eine Dispersion ist, wobei das Rhodoxanthin in einer Matrix aus modifizierter Lebensmittelstärke verkapselt ist und wobei die Dispersion ferner entweder Glycerin oder ein Saccharid umfasst.

**Revendications**

**1.** Boisson comprenant une forme de rhodoxanthine broyée ayant une taille de particules moyenne D(v,0,5) de la rhodoxanthine broyée dans la forme dans la plage allant de 400 à 600 nm, plus préférablement dans la plage allant de 500 nm à 600 nm, où la taille de particules moyenne est mesurée par Diffraction Laser ; Malvern Mastersizer 3000, distribution volumique par MIE.

**2.** Boisson selon la revendication 1, où la boisson possède une valeur chromatique b* dans la plage allant de -7,5 à 0 sur l'échelle de couleur CIELAB, préférablement une valeur chromatique b* dans la plage allant de -2,5 à 0 sur l'échelle de couleur CIELAB, plus préférablement une valeur chromatique b* dans la plage allant de -2,0 à -0,5 sur l'échelle de couleur CIELAB.

**3.** Boisson selon la revendication 1 et/ou 2, où la boisson possède une nuance h dans la plage allant de 350 à 360 sur l'échelle de couleur CIELAB, préférablement une nuance h dans la plage allant de 352 à 356 sur l'échelle de couleur CIELAB, plus préférablement une nuance h dans la plage allant de 353 à 355 sur l'échelle de couleur CIELAB.

**4.** Boisson selon l'une ou plusieurs quelconques parmi les revendications précédentes, où la quantité de rhodoxanthine broyée se trouve dans la plage allant de 1 ppm à 20 ppm, préférablement dans la plage allant de 1 à 15 ppm, plus préférablement dans la plage allant de 1 à 10 ppm, sur la base du poids total de la boisson.

**5.** Boisson selon l'une ou plusieurs quelconques parmi les revendications précédentes, où la boisson est choisie dans le groupe constitué par les boissons non alcoolisées, les eaux aromatisées, les eaux fortifiées, les boissons pour sportifs, les boissons minérales, les boissons gazeuses, les jus de fruits, les boissons non alcoolisées contenant du jus de fruits, les boissons alcoolisées, les boissons contenant du sucre et les boissons diététiques.

**6.** Boisson selon l'une ou plusieurs quelconques parmi les revendications précédentes, où la rhodoxanthine broyée est ajoutée à la boisson sous forme de dispersion où la rhodoxanthine broyée est encapsulée dans une matrice d'amidon alimentaire modifié.

**7.** Forme comprenant de la rhodoxanthine broyée, où la rhodoxanthine broyée dans la forme possède une taille de particules moyenne dans la plage allant de 400 à 650 nm, plus préférablement dans la plage allant de 500 nm à 600 nm, mesurée par Diffraction Laser ; Malvern Mastersizer 3000, distribution volumique par MIE.

**8.** Forme selon la revendication 7, qui est une dispersion.

**9.** Forme selon la revendication 7 et/ou 8, où la rhodoxanthine est encapsulée dans une matrice d'amidon alimentaire modifié.

**10.** Forme selon la revendication 9, comprenant en outre du glycérol ou bien un saccharide.

**11.** Procédé de fabrication d'une dispersion selon l'une ou plusieurs quelconques parmi les revendications 8 à 10, comprenant les étapes suivantes :

a) la fourniture d'une dispersion comprenant de la rhodoxanthine cristalline, de l'amidon alimentaire modifié, de l'eau et du glycérol ou un saccharide ;
b) le broyage de la dispersion telle qu'obtenue dans l'étape a) jusqu'à ce que la rhodoxanthine broyée dans la dispersion possède une taille de particules moyenne D(v,0,5) dans la plage allant de 400 à 650 nm, plus préférablement dans la plage allant de 500 nm à 600 nm, mesurée par Diffraction Laser ; Malvern Mastersizer 3000, distribution volumique par MIE.

**12.** Utilisation d'une forme de rhodoxanthine broyée ayant une taille de particules moyenne D(v,0,5) de la rhodoxanthine broyée dans la forme dans la plage allant de 400 à 600 nm, plus préférablement dans la plage allant de 500 nm à 600 nm, où la taille de particules moyenne est mesurée par Diffraction Laser ; Malvern Mastersizer 3000, distribution volumique par MIE, pour la coloration de boissons.

**13.** Utilisation selon la revendication 12, où la forme de rhodoxanthine broyée est une dispersion, où la rhodoxanthine est encapsulée dans une matrice d'amidon alimentaire modifié, et où la dispersion comprend en outre du glycérol ou bien un saccharide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2644041 A1
- EP 077439 A
- EP 085763 A


### Non-patent literature cited in the description


- **MERZLYAK et al.** *Photochem Photobiol Sci,* 2005, vol. 4, 333-340
- Modified Starches: Properties and Uses. CRC Press, Inc, 1986